# EUROPEAN PATENT SPECIFICATION

(11) **EP 0 958 789 B1**
(45) Date of publication and mention of the grant of the patent: **04.05.2005**
(21) Application number: 99109800.5
(22) Date of filing: 19.05.1999
(51) Int. Cl.: A61B 17/56, A61M 37/00, A61B 17/86, A61L 31/16

(54) **Apparatus for delivering antibiotic powders into the femoral canal**
Gerät zum Einführen antibiotischer Pulver in den Femurmarkkanal
Appareil pour délivrer des poudres antibiotiques dans le canal médullaire fémoral

(30) Priority: 20.05.1998 US 81963
(43) Date of publication of application: 24.11.1999
(73) Proprietor: Howmedica Osteonics Corp., Mahwah, NJ 07430 (US)
(72) Inventor: Scott, Christopher P., Hackensack, NJ 07601 (US)
(74) Representative: Hammond, Andrew David

(56) References cited:
- EP-A- 0 436 353
- DE-A- 4 216 496
- US-A- 4 653 489
- US-A- 4 863 444
- US-A- 5 433 718
- US-A- 5 681 289

## Description

### TECHNICAL FIELD

The invention relates to apparatus for dispensing medication at a surgical site. More particularly, the invention relates to apparatus for dispensing antibiotics at an orthopaedic surgical site.

### BACKGROUND OF THE INVENTION

In open surgical procedures, it is common to apply an antibiotic, analgesic, growth stimulator, or other chemical agent at the surgical site prior to closing the incision in order to control infection, decrease pain, promote growth, etc.

One of the most devastating complications of total joint arthroplasty is deep sepsis. Treatment of an infected joint replacement is difficult due to its location, and localized devascularization resulting from this procedure.

Current approaches to therapy for deep infections include systemic or parenteral antibiotic regimes, and the use of antibiotic impregnated acrylic bone cement. Due to the localized devascularization it is difficult to achieve therapeutic levels in the bone surrounding the implant without exceeding toxic serum concentrations when utilizing systemic or parenteral treatments. The use of antibiotic containing bone cement results in high local concentrations, while avoiding toxic serum levels, but the antibiotic has been shown to elute in trace quantities for extended periods of time (greater than one year). Residual trace amounts of antibiotics have raised concerns of resistant strain formation. An additional concern regarding adding antibiotics to bone cement is the possible degradation of mechanical properties of the bone cement whose primary function is as a fixation material.

According to the state of the art, it is preferable to apply therapeutic concentrations at the surgical site for a period of 7 to 10 days, with no residual antibiotics lingering for extended periods of time. It is also desirable to achieve these high local concentrations without elevating serum concentrations, thus reducing the threat of systemic toxicity. A means of antibiotic treatment that can be utilized for joint replacement procedures that either involve the use of bone cement or not is desirable as well.

U.S. Patent Number 5,681,289 to Wilcox et al. discloses a dispensing bladder for passing a low volume flow of a liquid chemical agent at an orthopaedic surgical site. The bladder is installed adjacent to or as part of an orthopaedic implant. It is coupled to a tube which receives a supply of liquid chemical such as an antibiotic via an injection port or an implanted or external reservoir and pump.

The bladder may be biodegradable so as to avoid the need for extensive surgery to remove it. However, the tube, injection site, pump, and reservoir must be surgically removed. Moreover, it is believed that the delivery of a liquid antibiotic in the femoral canal may degrade the mechanical properties of bone cement on an implant stem.

U.S. Patent No. 5 433 718 relates to an antibiotic eluding intramedullary nail apparatus. In one embodiment, the apparatus comprises bone stabilizing means in the form of an apertured tube. An antibiotic compound is disposed within the tube. Once inserted into the medullary canal, the antibiotic compound will exude from the tube via the apertures.

EP-A-0 436 353 relates to apparatus for aerosol administration of medication using an endotracheal tube.

### SUMMARY OF THE INVENTION

It is therefore an object of the invention to provide apparatus for delivering an antibiotic to an orthopaedic surgical site.

It is also an object of the invention to provide apparatus for delivering an antibiotic to an orthopaedic surgical site which does not require an additional implant which must subsequently be surgically removed.

It is another object of the invention to provide apparatus for delivering an antibiotic to an orthopaedic surgical site which will deliver a therapeutic dose of antibiotic over a predefined dosing period, for example 7-10 days.

It is still another object of the invention to provide apparatus for delivering an antibiotic to an orthopaedic surgical site which will not interfere with the mechanical properties of bone cement.

Another object of the invention is to provide apparatus for delivering an antibiotic to an orthopaedic surgical site which does not leave long term residual antibiotics at the site after the dosing period.

Another object of the invention is to provide apparatus for delivering an antibiotic to an orthopaedic surgical site which do not significantly elevate serum levels.

In accord with these objects which will be discussed in detail below, the apparatus of the present invention as claimed in claims 1 and 12 includes means for encapsulating the antibiotic in fine particles of bioabsorbable polymer which will yield a constant dissolution of the antibiotic over a predefined dosing period (such as a 7-10 day period), with residual antibiotic being thereafter delivered only until the bioabsorable polymer is resorbed; and means for delivering the fine particle powder to the surgical site as an aerosol spray.

It is preferable that the antibiotic and the polymer be thermostable so that the exothermic reaction of bone cement does not degrade the polymer or the antibiotic.

A suitable antibiotic for this purpose is an aminoglycoside and a suitable polymer is poly lactide-coglycolide (PLGA).

In situations where themostability is not an issue, for example where bone cement is not used, then virtually any antibiotic may be delivered via the techniques taught herein, such as (without limitation), penicillin, etc. Furthermore, those skilled in the art will readily appreciate that other bioabsorable polymers can be readily substituted for PLGA, for example (without limitation), PLA, etc.

One apparatus according to the invention includes a porous delivery tube which is dimensioned to fit comfortably in a bone canal and which is coupled to a aerosol canister containing the antibiotic powder of the invention.

Another apparatus according to the invention includes a porous delivery tube which is pre-loaded with measured amounts of antibiotic powder and which is adapted to be coupled to a source of compressed air.

Additional objects and advantages of the invention will become apparent to those skilled in the art upon reference to the detailed description taken in conjunction with the provided figures.

### BRIEF DESCRIPTION OF THE DRAWINGS

- FIG. 1: is a schematic sectional view of a first embodiment of an antibiotic delivery apparatus according to the invention;
- FIG. 2: is a schematic perspective view of a second embodiment of an antibiotic delivery apparatus according to the invention; and
- FIG. 3: is an enlarged perspective view of a component of the antibiotic delivery apparatus of FIG. 2.

### DETAILED DESCRIPTION

According to the invention, a powdered antibiotic is manufactured by encapsulating antibiotic in fine particles of bioabsorbable polymer.

Suitable processes for encapsulating an antibiotic in a resorbable powder are disclosed in the following nine journal articles,
(a) Van Hamont, J.E. et al., "Evaluation of Solvent Extraction and Solvent Evaporation Procedures for Production of Tobramycin-Releasing Microspheres," Proceed. Intern. Symp. Control. Rel. Bioact. Mater., 23 (1996), Controlled Release Society, Inc.
(b) Martinez, B. et al., "Tetracine Release from Biodegradable Microspheres," Proceed. Intern. Symp. Control. Rel. Bioact. Mater., 27 (1997), Controlled Release Society, Inc.
(c) Kim, J.H. et al., "The Modified Solvent Extraction Method on the Preparation of Poly(L-Lactic Acid) Microspheres," Proceed. Intern. Symp. Control. Rel. Bioact. Mater., 24 (1997), Controlled Release Society, Inc.
(d) Bittner, B. et al., "Preparation of Protein-Loaded Poly(Lactide-Co-Glycolide) Microspheres Using an Ultrasonic Atomizer." Proceed. Intern. Symp. Control. Rel. BlioaCt. Mater., 24 (1997), Controlled Release Society, Inc.
(e) Takada, S., "Novel Microencapsulation Technique for Controlled Release of a Water-Soluble Non-Basic Drug," Proceed. Intern. Symp. Control. Rel. Bioact. Mater., 23 (1996), Controlled Release Society, Inc.
(f) Jeyanthi, R. et al., "Novel Burst Free Programmable Biodegradable Microspheres for Controlled Release of Polypeptides," Proceed. Intern. Symp. Control. Rel. Bioact. Mater., 23 (1996), Controlled Release Society, Inc.
(g) Witschi, C., "Influence of the Preparation Method and Polymer Composition on Peptide Adsorption and Peptide Release from Biodegradable Microparticles During In Vitro Release," Proceed. Intern. Symp. Control. Rel. Bioact. Mater., 24 (1997), Controlled Release Society, Inc.
(h) Van Hamont, J.E. et al., "Effect of PLGA End Groups on Encapsulation and Release of Tobramycin from Microspheres Produced by Organic Processes," Proceed. Intern. Symp. Control. Rel. Bioact. Mater., 24 (1997), Controlled Release Society, Inc.
(i) Grandfils, C. et al., "Control of the Biodegradation Rate of Poly(DL-Lactide) Microparticles Intended as Chemoembolization Materials," Journal of Controlled Release 38 (1966).

The prepared antibiotic powder, when placed at a surgical site will yield a constant dissolution of the antibiotic over a period of 7-10 days.

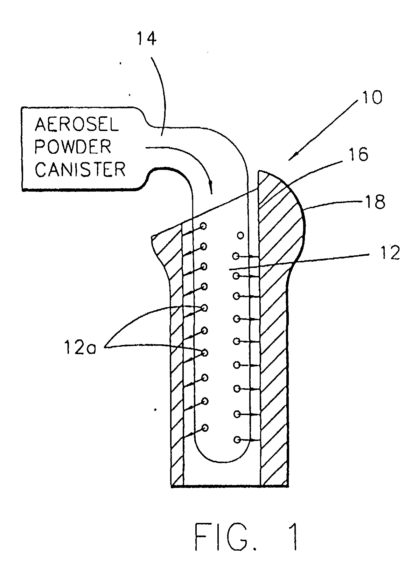

Turning now to FIG. 1, an apparatus 10 according to the invention for delivering the powdered antibiotic to an orthopaedic surgical site includes a porous tube 12 which is coupled to an aerosol container 14 which contains a measured amount of the powdered antibiotic for a single procedure.

According to a presently preferred illustrative embodiment, the tube 12 is dimensioned to fit comfortable in the intermedullary canal 16 of the femur 18. The tube 12 is made of a biocompatible material such as plastic or stainless steel and is made porous by perforations 12a which are evenly spaced about the tube 12.

The apparatus is used during an orthopaedic procedure after the femoral canal 16 is prepared, before the prosthetic implant is installed.

The apparatus 10 is operated by inserting the tube 12 into the IM canal 16 of the femur 18 and opening a valve (not shown) on the aerosol container 14.

The aerosol forces the antibiotic powder into the tube 12 and out through the perforations 12a into the wall of the IM canal 16.

According to a presently preferred embodiment, the aerosol container 14 contains enough powder and propellant for one application and is disposable after one use. After the aerosol is depleted, the apparatus 10 is removed from the femur and the prosthetic implant is installed in a conventional manner.

The antibiotic powder which was delivered to the IM canal will not interfere with bone cement, will dissolve a therapeutic level of antibiotic into the femur continuously over 7-10 days and will not leave long time residual antibiotics in the femur.

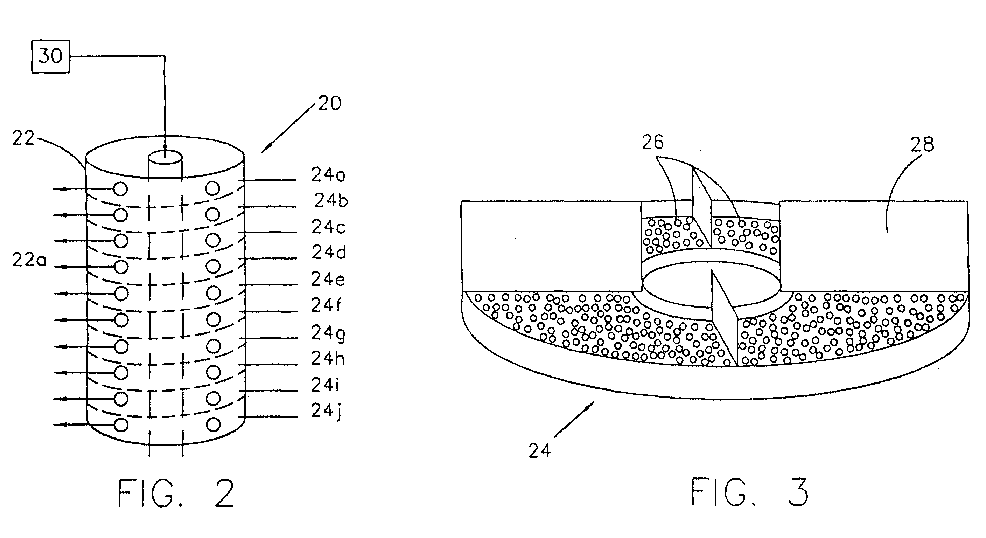

Turning now to FIGS. 2 and 3, another apparatus 20 according to the invention includes a porous delivery tube 22 which is pre-loaded with measured amounts of antibiotic powder 26 and which is adapted to be coupled to a source of compressed air 30.

The tube 22 may be made of biocompatible plastic or stainless steel which may be made porous by perforations 22a which are evenly spaced along the length of the tube.

According to this illustrative embodiment of the invention, a plurality of annular shelves 24 (e.g., 24a-24j) are arranged inside the tube 22 axially spaced apart from each other as shown in FIG. 2. Each shelf 24 carries a measured amount of antibiotic powder 26 as shown in FIG. 3.

According to a preferred embodiment, each shelf 24 has a plurality of orthogonal fins 28 which aid in stacking the shelves inside the tube 22 and directing the flow of powder 26 out of the perforations 22a.

The apparatus 20 is operated by inserting the tube 22 into the IM canal of the femur, attaching a source 30 of compressed air to the tube and forcing compressed air into the tube. The compressed air forces the antibiotic powder 26 off of the shelves 24 and out through the perforations 22a into the wall of the IM canal.

According to a presently preferred embodiment, the shelves 24 contain enough powder 26 for one application and the apparatus 20 is disposable after one use. After a measured period of dispensing, the apparatus 20 is removed from the femur and the prosthetic implant is installed in a conventional manner.

The antibiotic powder which was delivered to the IM canal will not interfere with bone cement, will dissolve a therapeutic level of antibiotic into the femur continuously over 7-10 days and will not leave long time residual antibiotics in the femur.

There have been described and illustrated herein several embodiments of a method and apparatus for delivering antibiotic powder to the IM canal of the femur.

While particular embodiments of the invention have been described, it is not intended that the invention be limited thereto, as it is intended that the invention be as broad in scope as the art will allow and that the specification be read likewise. Thus, while particular powders have been disclosed, it will be appreciated that other powders could be utilized. Also, while the invention has been disclosed for use in the IM canal of the femur, it will be understood that the invention may have utility in dispensing medication in other bone canals.

It will therefore be appreciated by those skilled in the art that yet other modifications could be made to the provided invention without deviating from its scope as so claimed.

## Claims

1. An apparatus (10; 20) for dispensing medication into a bone canal (16), said apparatus comprising:
(a) a porous tube (12; 22) dimensioned to fit into the bone canal (16); and
(b) a medication deliverable through said porous tube (12; 22),
**characterized in that**
(c) said medication is powdered, and
(d) that said apparatus further comprises fluid pressure means (14; 30) in the form of an aerosol container coupled to said tube for forcing said powdered medication through said porous tube.

2. An apparatus according to claim 1, wherein said powdered medication is contained within said aerosol container.

3. An apparatus according to claim 1 or 2, wherein said powdered medication (26) is contained within said porous tube (22) and said aerosol container is detachable from said porous tube.

4. An apparatus according to any one of claims 1 to 3, wherein said porous tube (22) includes a plurality of annular shelves (24) and said powdered medication (26) is contained on said shelves.

5. An apparatus according to claim 4, wherein each of said annular shelves (24) has a plurality of orthogonal vanes (28).

6. An apparatus according to any one of the preceding claims, wherein said powdered medication is an antibiotic encapsulated in a bioabsorbable polymer in such a way as to yield constant dissolution over a predefined therapeutic dosing period with residual antibiotic being delivered thereafter only until said bioabsorbable polymer is resorbed.

7. Apparatus as set forth in claim 6, wherein said predefined therapeutic dosing period is 7-10 days.

8. An apparatus according to claim 1, wherein said powdered medication is thermostable when exposed to an exothermic reaction of orthopaedic bone cement.

9. An apparatus according to claim 7, wherein said antibiotic is an aminoglycoside.

10. An apparatus according to claim 7, wherein said bioabsorbable polymer is poly lactide-co-glycolide (PLGA).

11. An apparatus according to claim 1, wherein said porous tube (12; 22) is one of a perforate plastic tube and a perforate stainless steel tube.

12. An apparatus (20) for dispensing medication into a bone canal (16), said apparatus comprising:
(a) a porous tube (22) dimensioned to fit into the bone canal (16); and
(b) a medication deliverable through said porous tube (22),
**characterized in that**
(c) said medication is powdered and is disposed in a plurality of locations within said tube, and
(d) that said apparatus further comprises fluid coupling means for coupling said tube to a source of fluid pressure for forcing said powdered medication through said porous tube.

## Patentansprüche

1. Vorrichtung (10; 20) zur Abgabe eines Arzneimittels in einen Knochenkanal (16), wobei die Vorrichtung folgendes umfasst:
(a) ein poröses Rohr (12; 22), das in seinen Abmessungen an den Knochenkanal (16) angepasst ist; und
(b) ein Arzneimittel, das durch das poröse Rohr (12; 22) abgegeben werden kann,
**dadurch gekennzeichnet, dass**
(c) das Arzneimittel pulverisiert ist und
(d) die Vorrichtung ferner eine Fluid-Druckeinrichtung (14; 30) in Form eines an das Rohr angekuppelten Aerosolbehälters umfasst, um das pulverisierte Arzneimittel durch das poröse Rohr zu drücken.

2. Vorrichtung nach Anspruch 1, wobei das pulverisierte Arzneimittel im Aerosolbehälter enthalten ist.

3. Vorrichtung nach Anspruch 1 oder 2, wobei das pulverisierte Arzneimittel (26) im porösen Rohr (22) enthalten ist und der Aerosolbehälter vom porösen Rohr abnehmbar ist.

4. Vorrichtung nach einem der Ansprüche 1 bis 3, wobei das poröse Rohr (22) eine Mehrzahl von ringförmigen Fächern (24) umfasst und das pulverisierte Arzneimittel (26) auf den Fächern enthalten ist.

5. Vorrichtung nach Anspruch 4, wobei die ringförmigen Fächer (24) jeweils eine Mehrzahl von rechteckigen Rippen (28) aufweisen.

6. Vorrichtung nach einem der vorstehenden Ansprüche, wobei es sich bei dem pulverisierten Arzneimittel um ein Antibiotikum handelt, das so in einem bioresorbierbaren Polymeren eingekapselt ist, dass sich über eine vorbestimmte therapeutische Dosierungsperiode hinweg eine konstante Auflösung ergibt, wobei restliches Antibiotikum anschließend erst dann abgegeben wird, wenn das bioresorbierbare Polymere resorbiert worden ist.

7. Vorrichtung nach Anspruch 6, wobei die vorbestimmte therapeutische Dosierungsperiode sieben bis zehn Tage beträgt.

8. Vorrichtung nach Anspruch 1, wobei das pulverisierte Arzneimittel thermostabil ist, wenn es einer exothermen Reaktion eines orthopädischen Knochenzements ausgesetzt wird.

9. Vorrichtung nach Anspruch 7, wobei es sich bei dem Antibiotikum um ein Aminoglycosid handelt.

10. Vorrichtung nach Anspruch 7, wobei es sich bei dem bioresorbierbaren Polymeren um Polylactid-co-glycolid (PLGA) handelt.

11. Vorrichtung nach Anspruch 1, wobei es sich bei dem porösen Rohr (12; 22) um ein perforiertes Kunststoffrohr oder um ein perforiertes Rohr aus rostfreiem Stahl handelt.

12. Vorrichtung (20) zur Abgabe eines Arzneimittels in einen Knochenkanal (16), wobei die Vorrichtung folgendes umfasst:
(a) ein poröses Rohr (22), das in seinen Abmessungen an den Knochenkanal (16) angepasst ist; und
(b) ein Arzneimittel, das durch das poröse Rohr (22) abgegeben werden kann,
**dadurch gekennzeichnet, dass**
(c) das Arzneimittel pulverisiert ist und in einer Mehrzahl von Positionen innerhalb des Rohrs angeordnet ist und
(d) die Vorrichtung ferner eine Fluid-Kupplungseinrichtung zum Ankuppeln des Rohrs an eine Quelle eines Fluiddrucks umfasst, um das pulverisierte Arzneimittel durch das poröse Rohr zu drücken.

## Revendications

1. Appareil (10, 20) pour délivrer un traitement dans un canal osseux (16), ledit appareil comportant :
(a) un tube poreux (12, 22) dimensionné pour s'agencer dans le canal osseux (16), et
(b) un traitement délivrable par ledit tube poreux (12, 22)
**caractérisé en ce que**
(c) ledit traitement est en poudre, et
(d) ledit appareil comporte de plus des moyens de pression de fluide (14, 30) sous la forme d'un conteneur d'aérosol associé audit tube pour pousser ledit traitement en poudre à travers ledit tube poreux.

2. Appareil selon la revendication 1, dans lequel ledit traitement en poudre est contenu à l'intérieur dudit conteneur d'aérosol.

3. Appareil selon la revendication 1 ou 2, dans lequel ledit traitement en poudre (26) est contenu à l'intérieur dudit tube poreux (22), et ledit conteneur d'aérosol est détachable dudit tube poreux.

4. Appareil selon l'une quelconque des revendications 1 à 3, dans lequel ledit tube poreux (22) comporte une pluralité d'étagères annulaires (24), et ledit traitement en poudre (26) est contenu dans lesdites étagères.

5. Appareil selon la revendication 4, dans lequel chacune desdites étagères (24) a une pluralité d'aubes orthogonales (28).

6. Appareil selon l'une quelconque des revendications précédentes, dans lequel ledit traitement en poudre est un antibiotique encapsulé dans un polymère bioabsorbable de façon à produire une dissolution en continu pendant une période de dosage thérapeutique prédéfinie, l'antibiotique résiduel étant délivré par la suite seulement jusqu'à ce que ledit polymère absorbable soit résorbé.

7. Appareil selon la revendication 6, dans lequel ladite période de dosage thérapeutique prédéfinie est de 7 à 10 jours.

8. Appareil selon la revendication 1, dans lequel ledit traitement en poudre est thermostable lorsqu'exposé à une réaction exothermique de ciment osseux orthopédique.

9. Appareil selon la revendication 7, dans lequel ledit antibiotique est un aminoglycoside.

10. Appareil selon la revendication 7, dans lequel ledit polymère bioabsorbable est du poly (lactide-co-glycolide) acide (PLGA).

11. Appareil selon la revendication 1, dans lequel ledit tube poreux (12, 22) est un tube parmi un tube plastique perforé et un tube en acier inoxydable perforé.

12. Appareil (20) pour la délivrance d'un traitement dans un canal osseux (16), ledit appareil comportant :
(a) un tube poreux dimensionné pour s'agencer dans le canal osseux (16), et
(b) un traitement délivrable à travers ce tube poreux (22),
**caractérisé en ce que**
(c) ledit traitement est en poudre, et est disposé dans une pluralité d'endroits à l'intérieur dudit tube, et
(d) ledit appareil comporte de plus des moyens de couplage du fluide pour coupler ledit tube à une source de pression de fluide afin de pousser ledit traitement en poudre à travers ledit tube poreux.
